# EUROPÄISCHE PATENTANMELDUNG

(11) **EP 2 457 509 A1**
(43) Veröffentlichungstag der Anmeldung: **30.05.2012**
(21) Anmeldenummer: 11189539.7
(22) Anmeldetag: 17.11.2011
(51) Int. Cl.: A61B 5/145, A61B 5/1473, B82Y 15/00

(54) **Implantierbare Sensoreinheit**

(30) Priorität: 24.11.2010 US 416774 P
(71) Anmelder: BIOTRONIK SE & Co. KG, 12359 Berlin (DE)
(72) Erfinder: Bunge, Andreas, 04105 Leipzig (DE)
(74) Vertreter: Lindner-Vogt, Karin L.

(57) **Zusammenfassung**

Die Erfindung betrifft eine in einem Implantat einsatzstabile Sensoreinheit zum qualitativen und/oder quantitativen *in vivo* Bestimmung eines Analyten umfassend
(i) einen Verbindungsbereich (3) mit wenigstens einer Bindungsstelle für den Analyten (7), wobei der Verbindungsbereich im Falle der Bindung des Analyten an die Bindungsstelle eine räumliche Veränderung erfährt,
(ii) einen ersten fluoreszenzaktiven Bereich (1) und
(iii) einen zweiten fluoreszenzaktiven Bereich (5/9), wobei der erste fluoreszenzaktive Bereich (1) und der zweite fluoreszenzaktive Bereich (5/9) an den Verbindungsbereich (3) so gebunden sind, dass im Falle einer durch die Bindung des Analyten an den Verbindungsbereich (3) bewirkte räumliche Veränderung des Verbindungsbereiches (3) eine Veränderung des Abstandes des erste fluoreszenzaktiven Bereiches (1) und des zweiten fluoreszenzaktiven Bereiches (5/9) zueinander erfolgt, ohne dass die Bindungen der fluoreszenzaktiven Bereiche (1, 5/9) an den Verbindungsbereich gelöst werden und wobei einer oder beide der fluoreszenzaktiven Bereiche (1, 5/9) zu mehr als 60 Gew.% aus einem anorganischen Material besteht.

## Beschreibung

Die Erfindung betrifft eine in einem Implantat einsatzstabile Sensoreinheit zur qualitativen und/oder quantitativen *in vivo* Bestimmung eines Analyten, ein Implantat umfassend eine solche Sensoreinheit, ein Verfahren zum qualitativen oder quantitativen Bestimmen eines Analyten im Körper eines Menschen oder eines Tieres sowie die Verwendung einer entsprechenden Sensoreinheit bzw. eines entsprechenden Implantates zum qualitativen oder quantitativen Bestimmen eines Analyten.

Die Funktionsweise des menschlichen und des tierischen Körpers basiert auf einer Vielzahl von biochemischen Vorgängen, die sich durch das Auftreten von bestimmten Verbindungen im Körper jeweils qualitativ und quantitativ beschreiben lassen. So lässt beispielsweise über die Glukosekonzentration eine Vielzahl von Aussagen über den Stoffwechselzustand des betroffenen Körpers machen. Dabei basieren die metabolen und anabolen Vorgänge im Körper auf einer Vielzahl miteinander kommunizierender ausgewogener Gleichgewichtszustände. Stoffwechselerkrankungen führen dazu, dass ein Teil dieser Gleichgewichtszustände gestört ist. Therapeutisch ist es häufig möglich, durch Gabe bestimmter Wirkstoffe, die durch Stoffwechselkrankheiten (oder auch andere Krankheiten) verursachten unerwünschten Konzentrationen bestimmter Verbindungen im Körper im Sinne des Stoffwechselgleichgewichts zu korrigieren. Das bekannteste und wirtschaftlich relevanteste Beispiel für diese Situation ist die Glukosekonzentration im Blut und/oder Plasma im Falle einer Diabeteserkrankung. Durch richtig dosierte Gabe von Insulin ist es möglich, unerwünschte Glukoselevel auf erwünschte Level zu bringen. Dabei ist die Dosis des zu verabreichenden Insulins selbstverständlich abhängig von der zu verändernden Glukosekonzentration. Daher ist es wünschenswert, möglichst in Echtzeit Informationen über den jeweiligen Glukosespiegel zu gewinnen. Entsprechendes gilt auch für andere Verbindungen (Analyten) als Glukose, wie zum Beispiel Hormone, Elektrolyte, Lipide, Aminosäuren, Peptide, Proteine, Neurotransmitter und Stoffwechselendprodukte.

In der Literatur werden verschiedene Methoden zur kontinuierlichen optischen in vitro Konzentrationsbestimmung von Analyten beschrieben. Primär steht hier für diesen Text die Messung der Fluoreszenz im Vordergrund:
Eine Methode zur Bestimmung der Konzentration von Glukose nach Pickup et al. / Biosensors and Bioelectronics 20 (2005) 1897-1902, basiert auf einer Verdrängungsreaktion. Hierfür wird das Protein Concavalin A (Con A) eingesetzt, das unter physiologischen Bedingungen als Tetramer 4 Bindungsstellen für Glukose besitzt. Das Con A ist kovalent mit einem organischen Fluoreszenzfarbstoff (APC) versehen. Im glukosefreien Zustand sind an die 4 Bindungsstellen fluoreszenzmarkierte Dextranmoleküle (fluoreszente Polymere bestehend aus Glukose und Fluorophor) gebunden. Die Assoziation der fluoreszenzmarkierten Dextranmoleküle mit dem fluoreszenten Con A kann über den Fluoreszenz Resonanz Energie Transfer (FRET) nachgewiesen werden. FRET basiert auf der Energieübertragung ausgehend von einem Donor-Fluorophor auf einen benachbarten fluoreszierenden Akzeptor und kann über eine Abnahme der Donorfluoreszenz und/oder Zunahme der Akzeptorfluoreszenz detektiert werden. Befindet sich Glukose in der Probenlösung, dann kommt es zur Verdrängung der gebundenen markierten Dextranmoleküle durch die Glukose. Hierdurch befinden sich weniger fluoreszente Dextranmoleküle in der räumlichen Nachbarschaft der Con A, was sich in einer Reduktion des FRETs bemerkbar macht.

Eine weitere Methode nach Ye et al., Genetic Engineering of an Allosterically Based Glucose Indicator Protein for Continous Glucose Monitoring by Fluorescence Resonance Energy Transfer /Anal. Chem. 2003, 75, 3451-3459, zur Bestimmung der Glukosekonzentration basiert auf der Konformationsänderung des Glukosebindungsproteins (GBP), welches in Bakterien wichtige Funktionen übernimmt. Das GBP stellt bzgl. der Struktur eine Art Scharnier dar, an dessen Drehpunkt die Glukose selektiv binden kann. Durch Bindung der Glukose kommt es zur Konformationsänderung des GBPs. Der Nachweis erfolgt auch hier über FRET. Hierfür werden an unterschiedliche Positionen des GBP-Moleküls fluoreszente Moleküle, spezielle Proteine gebunden. Im glukosefreien Zustand hat die kompakte dreidimensionale Struktur des GBPs zur Folge, dass die beiden fluoreszenten organischen Molekülteile in einem kurzen Abstand voneinander entfernt lokalisiert sind, der FRET ist groß. Liegt Glukose in der Probenlösung vor, bindet Glukose an das GBP, ein reduzierter FRET kann aufgrund der Konformationsänderung des GBP und des somit größeren Abstands der fluoreszenten Proteine beobachtet werden. Bis zur Konzentration, bei der alle GBPs mit Glukose abgesättigt sind, verändert sich der FRET in Abhängigkeit von der Glukosekonzentration.

Alternativ wurde in der Literatur (Medintz et al, Self-assembled nanoscale biosensors based on quantum dot FRET donors, nature materials Vol. 2, September 2003, pages 630 - 638) eine Methode zur Konzentrationsbestimmung eines Zweifachzuckers auf Grundlage von anorganischen fluoreszenten Nanopartikeln, den so genannten Quantum Dots (QDs), berichtet. QDs besitzen gegenüber den meistens eingesetzten organischen Fluoreszenzfarbstoffen wie Rhodamin, NBD oder speziellen Proteinen folgende interessante Eigenschaften: Sie zeichnen sich durch eine hohe Photostabilität und vergleichsweise hohe Helligkeit aus. Ihre herausragenden optischen Eigenschaften lassen sich sehr einfach für die benötigte Anwendung anpassen, indem ihre Größe während der Synthese eingestellt wird. Der Nachweis des Zweifachzuckers Maltose basiert auf einer Verdrängungsreaktion. Das Erkennungselement stellt hier das Maltosebindungsprotein (MBP) dar. Dieses Molekül kann jedoch aufgrund der hohen Selektivität für Maltose keinen Einfachzucker (Glukose) binden. Für den optischen Nachweis ist an das MBP ein Quantum Dot gebunden. Die Methodik benötigt ein weiteres Reagenz, ein Cyclodextrin (zyklisches Zuckermolekül) mit kovalent gebundenem Fluoreszenzquencher (QSY-9). In Abwesenheit von Maltose ist das Cyclodextrin an das MBP gebunden, der Fluoreszenzquencher löscht die Fluoreszenz des Quantum Dots. Befindet sich Maltose in der Probenlösung, dann kommt es zur Verdrängung des Cyclodextrins durch die Maltose, die Fluoreszenz wird weniger stark gelöscht und steigt an. Mittels Detektion der Fluoreszenz, die von der Maltosekonzentration abhängig ist, kann auf die Konzentration der Maltose geschlossen werden.

In der Literatur wird ebenfalls ein Nachweisystem für Kalzium (Ca²⁺) diskutiert (Miyawaki et al., Fluoreschent indicator for Ca²⁺ based on green fluorescent proteins and calmodulin). Im Mittelpunkt steht hier das selektiv-Ca²⁺-bindende Calmodulin, das an 2 Positionen mit fluoreszenten Proteinen modifiziert ist. Dieses Konstrukt wird in der Veröffentlichung dazu genutzt, die Kalziumkonzentration innerhalb von Zellen zu bestimmen.

Der Nachteil an den beschriebenen Methoden ist darin zu sehen, dass keine der oben beschriebenen Lösungen geeignet ist, im menschlichen oder tierischen Körper dauerhaft eingesetzt zu werden. Zum Teil verbietet sich sogar ein Einsatz entsprechender Sensoreinheiten im Körper, wie im Falle der Variante mit dem Einsatz des Fluoreszenzquenchers QSY-9 vgl. oben, Medinsk et al.: Hier können Moleküle in den Körper gelangen (hier der Fluosenzquencher) deren Auswirkung auf den Körper unbekannt sind. Darüberhinaus sind die beschriebenen Systeme *in vivo* nicht stabil, insbesondere solche, die auf Verdrängungsreaktionen beruhen, da hier stets beim Austausch mit Körperflüssigkeiten entsprechende Reagenzien nachzudosieren sind. Darüber hinaus sind die Fluorophore zum Teil unter Bedingungen im Körper nicht ausreichend stabil.

Aufgabe der vorliegenden Erfindung war es daher, eine Sensoreinheit zur qualitativen und/oder quantitativen *in vivo* Bestimmung eines Analyten anzugeben, die unter *in vivo* Bedingungen einsatzstabil ist und insbesondere keine zusätzlichen Reagenzien benötigen, die leicht in den Körper gelangen können und diesen potentiell belasten. Die Sensoreinheit sollte optische Signale geben können, wobei Ihre Komponenten eine hohe Photostabilität und Quantenausbeute besitzen sollten. Ferner war bevorzugt, eine möglichst geringe pH-Wertabhängigkeit der Sensoreinheit zu erreichen.

Erfindungsgemäß wird diese Aufgabe gelöst durch eine im Implantat einsatzstabile Sensoreinheit zum qualitativen und/oder quantitativen *in vivo* Bestimmung eines Analyten umfassend
(i) einen Verbindungsbereich (3) mit wenigstens einer Bindungsstelle für den Analyten (7),
   wobei der Verbindungsbereich im Falle der Bindung des Analyten an die Bindungsstelle eine räumliche Veränderung erfährt,
(ii) einen ersten fluoreszenzaktiven Bereich (1) und
(iii) einen zweiten fluoreszenzaktiven Bereich (5/9),
wobei der erste fluoreszenzaktive Bereich (1) und der zweite fluoreszenzaktive Bereich (5/9) an den Verbindungsbereich (3) so gebunden sind, dass im Falle einer durch die Bindung des Analyten an den Verbindungsbereich (3) bewirkte räumliche Veränderung des Verbindungsbereiches (3) eine Veränderung des Abstandes des erste fluoreszenzaktiven Bereiches (1) und des zweiten fluoreszenzaktiven Bereiches (5/9) zueinander erfolgt, ohne dass die Bindungen der fluoreszenzaktiven Bereiche (1, 5/9) an den Verbindungsbereich gelöst werden und wobei einer oder beide der fluoreszenzaktiven Bereiche (1, 5/9) zu ≥ 60 Gew.%, bevorzugt ≥ 70 Gew.% aus einem anorganischen Material besteht.

Im Implantat einsatzstabil im Sinne der vorliegenden Erfindung bedeutet, dass die Sensoreinheit über einen Zeitraum von ≥ dreißig Tagen, bevorzugt ≥ sechzig Tagen weiter bevorzugt ≥ hundert Tagen, weiter bevorzugt ≥ hundertfünfzig Tagen und besonders bevorzugt ≥ einem Jahr wenigstens zu 80% in der ursprünglichen Signalstärke im implantierten Zustand und bei potentiellem Kontakt mit dem jeweiligen Analyten bei identischer Konzentration des Analyten zu erzeugen vermag. Hierbei ist dem Fachmann selbstverständlich klar, dass eine entsprechende Aussage statistisch signifikant natürlich nur für eine Vielzahl von (Einzel-) Sensoreinheiten gewonnen werden kann. Im Sinne der vorliegenden Beschreibung ist dabei die ursprüngliche Signalstärke der Wert, den die Sensoreinheit bei gegebener Konstellation unmittelbar nach Implantation geliefert hat.

Als Verbindungsbereich ist grundsätzlich jedes Molekül denkbar, das eine die Verbindungsstelle für den Analyten umfasst und das bei Eintreten des Bindungsereignisses eine räumliche Veränderung erfährt. Ein Beispiel für eine solche räumliche Veränderung ist beispielsweise eine Konformationsänderung eines Proteins. Dementsprechend sind bevorzugte Verbindungsbereiche auch Proteine. Bevorzugte den Analyten selektiv bindende Moleküle sind zum einen Glykoproteine, wie z.B. der Glukosetransporter des Bakterienstamms E.coli oder der humane Glukoserezeptor, zum anderen Calmodulin bzw. jeweils Fragmente oder Derivate davon sowie molekular designte bzw. molekular geprägte Moleküle. Darüber hinaus kann das Bindungsereignis über Wirt-Gast-Komplexe, z.B. durch ionenselektive Kronenether und Kryptanden bzw. über Valinomycin oder andere Ionophore vermittelt werden.

Ein fluoreszenzaktiver Bereich im Sinne der vorliegenden Erfindung ist ein Bereich, der Fluoreszenz bewirken kann (Fluorophor) oder Fluoreszenz löschen kann (Quencher). Im Sinne der Erfindung ist es selbstverständlich, dass mindestens einer der beiden fluoreszenzaktiven Bereiche ein Fluorophor sein muss. Die erfindungsgemäß einzusetzenden fluoreszenzaktiven Bereiche bestehen dabei zu erheblichen Anteilen aus anorganischen Materialien, was positiv ihre Fluoreszenzeigenschaften und die Stabilität beeinflussen kann. Dabei ist mit dem "fluoreszenzaktiven Bereich" im Zusammenhang mit der Angabe von Gewichtsprozent an anorganischem Material tatsächlich nur der Bereich gemeint, der an dem Fluoreszenzereignis, also dem Aussenden und Empfangen entsprechender Quanten, beteiligt ist. Explizit nicht in die Berechnung des relativen Anteils an anorganischem Material geht eine eventuelle Beschichtung eines "Fluoreszenzkernes" ein.

Bevorzugt besteht im Sinne der vorliegenden Definition der fluoreszenzaktive Bereich zu ≥ 85 Gew.%, weiter bevorzugt zu ≥ 90 Gew.%, weiter bevorzugt ≥ 95 Gew.%, besonders bevorzugt zu 100% aus anorganischem Material.

Das Nachweissystem basiert auf der selektiven, spezifischen reversiblen Bindung des Analyten an ein sensitives Molekül (die Sensoreinheit). Aufgrund der Änderung der räumlichen Struktur des sensitiven Moleküls durch die Bindung des Analyten kommt es zur Veränderung des Abstandes zwischen dem wenigstens teilweise anorganischen Fluorophor (erster fluoreszenzaktiver Bereich) und einem Quencher bzw. einen zweiten bevorzugt ebenfalls wenigstens teilweise anorganischen Fluorophor (jeweils zweiter fluoreszenzaktiver Bereich). Beide sind erfindungsgemäß fester Bestandteil der Sensoreinheit.

Das durch die erfindungsgemäße Sensoreinheit erzeugte Fluoreszenzsignal wird dabei abhängig vom Bindungsereignis (dem Vorliegen einer oder mehrerer Bindungen des Analyten an die Sensoreinheit bzw. dem Nicht-Vorliegen entsprechender Bindungen) moduliert. Bevorzugte Modulationen in diesem Zusammenhang sind die abstandsabhängige Fluoreszlöschung (Quench) oder der Fluoreszen-Resonanz-Energie-Tranfer (FRET) die zur Veränderung der Fluoreszenzintenesität oder der Fluoreszenzlebenszeit der anorganischen Fluoroforenabhängigkeit von Analytenkonzentrationen führen. Gegebenenfalls kann eine bevorzugte Modulation auch eine Wellenlängeverschiebung des während des Fluoreszenzereignisses abgegebenen Lichts gegenüber dem aufgenommenen Licht sein.. Die Analytenkonzentration kann dabei in Abhängigkeit von der Identität und/oder Lebensdauer des Fluoreszenzsignales mittels einer Kalibrationskurve ermittelt werden. Grundsätzlich sind auch rein qualitative Anwendungen denkbar, da bereits das Vorliegen bzw. Nichtvorliegen eines bestimmten Signales ausreichen kann, um ein Bindungsereignis qualitativ zu belegen.

Bevorzugt im Zusammenhang mit der vorliegenden Erfindung ist, dass die beiden fluoreszenzaktiven Bereiche vor dem Bindungsergeignis zueinander innerhalb des Förster Radius beabstandet sind und nach dem Bindungsereignis außerhalb des jeweiligen Förster Radius. Selbstverständlich ist je nach Ausgestaltung des Verbindungsbereiches auch der genau umgekehrte Fall denkbar, nämlich dass vor dem Bindungsereignis der erste fluoreszenzaktive Bereich und der zweite fluoreszenzaktive Bereich zueinander außerhalb des jeweiligen Förster Radius beabstandet sind und nach dem Bindungsereignis innerhalb des jeweiligen Förster Radius. Dementsprechend ist es bevorzugt, dass der Radius der fluoreszenzaktiven Bereiche zueinander im Bereich von 1 bis 10 nm, bevorzugt 2 bis 6 nm liegt für den Zustand, in dem das (stärkere) Fluoreszenzsignal erzeugt werden soll. Für den Zustand, in dem das schwächere Fluoreszenzsignal oder bevorzugt kein Fluoreszenzsignal erzeugt werden soll, ist der Abstand der beiden fluoreszenzaktiven Bereiche zueinander ≥ 4 nm bevorzugt ≥ 7 nm, weiter bevorzugt ≥ 10 nm und besonders bevorzugt 12 nm. Die in diesem Absatz gemachten Angaben gelten für die Situation, bei der FRET für die Signalerzeugung die entscheidende Rolle spielt.

Im Falle der Fluoreszenzlöschung sind die Abstände der beiden fluoreszenzaktiven Bereiche zueinander für den Fall, dass das (stärkere) Signal erzeugt werden soll, bevorzugt ≥ 2 nm, weiter bevorzugt ≥ 4 nm und besonders bevorzugt ≥ 6 nm und für den Fall dass das schwächere bzw. kein Signal erzeugt werden soll ≤ 6 nm, bevorzugt ≤ 2 nm und besonders bevorzugt ≤ 2 nm.

Der Vorteil der erfindungsgemäßen Sensoreinheit besteht zum einen in ihrer Stabilität: Durch den Einsatz ausgewählter anorganischer Materialen für wenigstens einen Fluorophor (vgl. auch weiter unten) lässt sich eine hohe Photostabilität und Quantenausbeute sowie eine geringe pH-Wert-Abhängigkeit der Fluoreszenz, die durch den erfindungsgemäßen Sensor erzeugt werden kann, erreichen. Überraschenderweise lässt sich damit das bisherige Stabilitätsproblem fluoreszenzbasierter Nachweissysteme, die implantiert *in vivo* Messwerte liefern sollen, überwinden. Gleichzeitig ist es nicht erforderlich, zusätzliche Reagenzien einzusetzen, die die Fluoreszenzeigenschaften beeinflussen (z.B. an den Sensor reversibel bindbare Quencher). Somit lässt sich die Sensoreinheit prinzipiell ohne dass körperfremde Verbindungen auf sie wirken für einen langen Zeitraum im Körper belassen. Außerdem benötigt die Sensoreinheit keine potentiell für den Körper schädlichen, lediglich reversibel gebundenen Reagenzien.

Bevorzugt ist eine erfindungsgemäße Sensoreinheit, wobei einer oder beide der fluoreszenzaktiven Bereiche ein gegebenenfalls beschichteter fluoreszenzaktiver Nanopartikel sind.

Weiter bevorzugt ist in diesem Zusammenhang, dass der Nanopartikel ausgewählt ist aus der Gruppe bestehend aus Quantum Dot, Nanophosphor und farbstoffmarkiertem Silikat.

Nanopartikel im Sinne der vorliegenden Erfindung sind Partikel in einer Größe von 1 nm bis 200 nm, jeweils bezogen auf den größten Durchmesser des Partikels, bevorzugt im Bereich von 3 bis 100 nm. Die Partikelgröße wird dabei bevorzugt mittels Lichtstreuung nach ISO 22412 ermittelt.

Insbesondere werden unter Quantum Dots (Quantenpunkten) nach vorliegender Erfindung fluoreszierende anorganische Nanoteilchen verstanden, die halbleitende Materialien sind und aus II-VI- oder III-V-Halbleitern bestehen und vorzugsweise eine Kernhüllenstruktur aufweisen. Dabei haben solche halbleitenden Materialien vorzugsweise einen anorganischen Kern von der Größe von vorzugsweise unter 10 nm (größter Durchmesser siehe oben). Bevorzugt sind solche Halbleiternanoteilchen gekappt, d. h. z. B. mit einer organischen Hülle umgeben. Zur Solubilisierung der Nanopartikel im wässrigen Medium ist es bevorzugt, dass die Oberfläche der Nanopartikel Strukturen mit funktionellen Schutzschichten modifiziert wird, ohne dass die optischen Eigenschaften verändert werden. Hierzu zählen die Modifikationen mit Thiol-Monoschichten, thiolierten Siloxanen, Glutathionen, Peptiden, Proteinen, Dendronen, Polyethylenglykol, Monosacchariden, Disacchariden, Trisacchariden, niedermolekulare Polysacchariden, hydrophile Vitaminen, lipophilen Vitaminen, Fettsäuren, Polyalkoholen, Teflon, Aminosäuren, unspezifischen Peptiden oder Proteinen, Phosphorylcholin, Polylaktat und/oder Derivaten der genannten Verbindungen. Weiterhin ist die Anbindung von langen hydrophoben Ketten denkbar, die zusammen mit Phospholipiden oder Diblockcopolymeren Bilayer bilden. Alternativ ist auch eine Beschichtung der Partikel mit Silica, Dentrimeren, amphiphilen Polysacchariden und Polymeren denkbar. An diese Oberflächenmodifikationen kann über physikalische Adsorption oder bevorzugt kovalent der Verbindungsbereich (das sensitive Molekül) gegebenenfalls auch über einen Linker gebunden werden.

Bevorzugte Quantum Dots in der vorliegenden Erfindung sind halbleitende Materialien ausgewählt aus der Gruppe bestehend aus InAs, InP, GaAs, GaP, GaN, InGaAs, GaInP/InP, CdO, CdSe, CdS₅CdTe ZnO, ZnS, ZnSe, ZnTe, PbS, PbSe, PbTe, HgS, HgSe und HgTe besonders bevorzugt CdSe, CdS oder CdTe.

Unter Nanophosphoren werden in vorliegender Anmeldung wiederum anorganische Nanoteilchen verstanden, die aus nicht leitenden oder halb-leitendenden Materialien bestehen und mit Ionen der Seltenen Erden dotiert sind. Vorzugsweise handelt es sich demnach um halbleitende Materialien. Prinzipiell können als Material für die dotierten Nanophosphoren folgende Verbindungen gewählt werden, wobei in der folgenden Schreibweise rechts vom Doppelpunkt ein oder mehrere Dotierelemente und links vom Doppelpunkt das Wirtsmaterial aufgeführt sind. Das Wirtsmaterial kann Verbindungen aus der Gruppe der Phosphate, Silikate, Germanate, Oxide, Sulfide, Oxysulfide, Selenide, Sulfoselenide, Vanadate, Niobate, Arsenate, Tantalate, Wolframate, Molybdate, Halogenate, Nitride, Borate, Aluminate, Gallate, und Halogenide sein. Besonders bevorzugt sind Nanophosphore, die auf Basis von Lanthanphosphat und/oder Yytrium-Vanadiumoxid hergestellt sind. Beispiele sind LiLEu; Al₂O₃:Eu; BaFCl:Sm; BaFBnEu; BaY₂F₈:Ln (Ln-Pr, Tm, Er, Ce), BaMgAl₁₆O₂₇=Eu; BaMgAl ₁₄O₂₃:Eu; BaMgAl₁₀O₎₇:Eu; BaMgAl₂O₄=Eu₁Ce(Mg₁Ba)Al₁₁Oi_{9;} MgAl₁ |O₁₉:Ce; (Mg,Ca)S:Eu; MgW0₄:Sm; CaS:Ln (Ln=Lanthanide); CaW0₄;Sm; CaSO₄:Ln (Ln=Lanthanide); SrS:Ln (Ln=Lanthanide) ,Sr₂P₂O₇; SrGa₂S₄:Ln (Ln=Lanthanide); YF₃ :Ln (Ln=Lanthanide); Y₂O:Ln (Ln=Lanthanide); Y(P,V)O₄:Eu; YOCl:Yb,Er; LuVO₄:Eu; GdVO₄:Eu; Gd₂O₂SjTb; GdMgB₅O₁₀: CesTb; LaOBr:Tb; La₂O₂S:Tb; LaF₃: Nd,Ce;BaYb₂F₈:Eu; NaYF₄: Yb₅Er; NaGd F₄: Yb₁Er; NaLaF₄: Yb₅Er; LaF₃: Yb₁Er₅Tm; BaYF₅ :Yb,Er; Ga₂O₃: Dy; GaN: A (A= Pr, Tm₅ Er, Ce); Gd₃Ga₅Oi₂=Tb; LiLuF₄: A (A-Pr₅ Tm₅ Er, Ce); CaSiO₃:Ln; CaS:Ln; CaO:Ln; ZnS:Ln; MgF₂:Ln mit Ln=Lanthanide YVO₄:Ln; LnPO₄:Ce,Tb; Y₂O₃:Ln; Y₂O₂S:Ln; Y₂Si0₅:Ln mit Ln=Lanthanide.

Vorzugsweise haben die Nanophosphore einen anorganischen Kern, der von einer organischen Hülle umgeben ist. Darüber hinaus ist bevorzugt, dass Nanophosphore einen anorganischen Kern von vorzugsweise einen größten Durchmesser unter 10 nm haben.

Das Emmisionsspektrum hängt hierbei von der eingesetzten Dotierung ab und nicht, wie bei den Quantum Dots, vom Durchmesser, die Größe der Nanophosphore muss bei der Synthese im Vergleich zu den Quantum Dots in nicht so engen Grenzen eingestellt werden. Vorteil der Nanophosphore ist ebenfalls die Photostabilität.

Alternativ können Silica-Nanopartikel eingesetzt werden, die mit Farbstoffen gefüllt sind, die so genannnten Dye-Doped Silica-Nanopartikel. Diese bleichen nicht aus, können aufgrund der äusseren Schicht gut im wässrigen Medium eingesetzt werden und ermöglichen eine einfache Ankopplung des Verbindungsbereiches.

Es kann ― wie bereits oben beschrieben ― bevorzugt sein, dass einer der fluoreszenzaktiven Bereiche ein Quencher ist. Bevorzugte Quencher im Zusammenhang mit dieser Erfindung sind Nanopartikel aus Metallen wie Gold und Silber, sowie Fullerene. Ebenfalls bevorzugt sind organische Quencher wie Doxyl, Mitglieder der DDQ-Familie, Dabcyl, Eclipse, Mitglieder der Iowa Black-Familie, Mitglieder der BHQ-Familie, Mitglieder der QSY-Familie, heterocyclische Chinonderivate und DABCYL. Diese Quencher haben sich im Sinne der Erfindung als besonders effektiv gezeigt.

Bevorzugt im Sinne der Erfindung ist eine erfindungsgemäße Sensoreinheit, wobei der Analyt ausgewählt ist aus der Gruppe bestehend aus Elektrolyten, Kohlehydraten, Metaboliten, Stoffwechselprodukten, Aminosäuren, Peptiden, Fetten, Fettsäuren, Lipiden, Proteinen, Neurotransmitter, Polyelektrolyten, Ribonukleinsäuren, Desoxyribonukleinsäuren, Nukleotiden, Hormonen und Wirkstoffen.

Im Speziellen können folgende Analyten bevorzugt sein: Albumine/Globuline, Alkalische Phosphatase, Alpha-1-Globulin, Alpha-2-Globulin, Alpha-1-Antitrypsin, Alpha-1-Fetoprotein, Alpha-Amylasen, Alpha-Hydroxybutyrat-Dehydrogenase, Ammoniak, Antithrombin III, Bicarbonat, Bilirubin, Blutkörperchen-Senkungsgeschwindigkeit, Blutungszeit, Carbohydrate-Antigen 19-9, Carcino-embryonales Antigen, Chlorid, Cholesterin, Cholinesterase, Cobalamin/Vitamin B 12, Coeruloplasmin, C-reaktives Protein, Cystatin C, D-Dimere, Eisen, Erythropoetin, Erythrozyten, Ferritin, Fetuin A Fibrinogen, Folsäure/Vitamin B9, Freies Tetrajodthyronin (fT4), Freies Trijodthyronin (fT3), Gamma-Glutamyltransferase, Glukose, Glutamat-Dehydrogenase, Glutamat-Oxalazetat-Transaminase, Glutamat-Pyruvat-Transaminase, Glykohämoglobin, Hämatokrit, Hämoglobin, Haptoglobin, Harnsäure, Harnstoff, HDL-Cholesterin, Homozystein, Immunglobulin A, Immunglobulin E, Immunglobulin G, Immunglobulin M, INR, Kalium, Kalzium, Kreatinin, Kreatin-Kinase, Kupfer, Laktat, Laktat-Dehydrogenase, LDL-Cholesterin, Leukozyten, Lipase, Lipoprotein, Magnesium, mittlere korpusukuläre Hämoglobin-Konzentration, mittleres korpuskuläres Hämoglobin, mittleres korspuskuläres Volumen, Myoglobin, Natrium, NT-proBNPBNP, Osmolalität, Phosphat, pH-Wert, Plasma-Thrombin-Gerinnungszeit, Prostataspezifisches Antigen, Retikulozyten, Rheumafaktor, Thrombozyten, Thyreoidea stimulierendes Hormon, Transferrin, Triglyzeride, Troponin T, Muskarinrezeptor-Antagonisten, neuromuskulär blockierende Stoffe, Cholesterinesterase-Hemmstoffe, Adrenozeptor-Agonisten, indirekt wirkende Sympathomimetika, Methylxanthine, alpha-Adrenorezeptor-Antagonisten, Mutterkornalkaloide, beta-Adrenozeptor-Antagonisten, Inaktivierungshemmstoffe, Antisympathonika, 5-HT-Rezeptor-agonisten, Histaminrezeptor-Agonisten, Hiastaminrezeptor-Antagonisten, Analgetika, Lokalanästhetika, Sedativa, Antikonvulsiva, Konvulsiva, Muskelrelaxantien, Antiparkinsonmittel, Neuroleptika, Antidepressiva, Lithium, Tranquillantien, Immunsuppressiva, Antirheumatika, Antiarrhythmika, Antibiotika, ACE-Hemmer, Aldosteronrezeptor-antagonisten, Diuretika, Vasodilatatoren, positiv inotrope Substanzen, antithrombotische/thrombolytische Substanzen, Laxantien, Antidiarrhoioka, Pharmake bei Adipositas, Uricostatika, Uricosurika, Lipidsenker, Antidiabetika, Anithypoglykämia, Hormone, Iodsalze, Threostatika, Eisen, Vitamine, Spurenelemente, Virostatika, Antimykotika, Antituberkulotika, Substanzen bei Tumorchemotherapie sowie deren Derivate.

Die erfindungsgemäße Sensoreinheit eignet sich besonders gut für den Nachweis der vorgenannten Analyten, da diese sich regelmäßig in Körperflüssigkeiten befinden. Die erfindungsgemäße Sensoreinheit eignet sich grundsätzlich besonders gut, um in Körperflüssigkeiten oder wenigstens in Kontakt mit Körperflüssigkeiten eingesetzt zu werden, da auf Basis der Flüssigkeit ein Transport und/oder Diffusion des jeweiligen Analyten zur Sensoreinheit gut gewährleistet wird.

Bevorzugt im Zusammenhang mit der Erfindung ist, dass der Analyt Glukose oder Kalzium ist.

Grundsätzlich ist der Aufbau der Sensoreinheit durch den Fachmann flexibel wählbar. Entscheidend ist, dass sowohl der erste fluoreszenzaktive Bereich als auch der zweite fluoreszenzaktive Bereich so an den Verbindungsbereich gebunden sind, dass dieser bei einer Bindung des Analyten an seine Bindungsstelle eine räumliche Veränderung erfährt, die wiederum Einfluss auf den Abstand der fluoreszenzaktiven Bereiche zueinander hat. Dabei kann die Bindung der fluoreszenzaktiven Bereiche wie bereits oben beschrieben zum Beispiel über einen Linker erfolgen, sie kann auch direkt an den C-Terminus und/oder N-Terminus des Verbindungsbereiches erfolgen (z. B. falls dieser ein Protein ist), sie kann aber ebenso an geeignete Seitenketten des Verbindungsbereiches vorgesehen werden.

Bestandteil der Erfindung ist auch ein Implantat, umfassend eine erfindungsgemäße Sensoreinheit. Dieses Implantat umfasst regelmäßig einen Außenbereich (ggf. Außenwände und/oder Membran), der den Kontakt der erfindungsgemäßen Sensoreinheit mit dem Körperumfeld einschränkt. Bevorzugt umfasst diese ein erfindungsgemäßes Implantat im Bereich des Außenbereiches eine semipermeable Membran, die selbstverständlich für den Analyten durchlässig ist.

Durch eine Einbringung der Sensoreinheit im Rahmen eines Implantates in den Körper ist es möglich, den Kontakt von Körperbestandteilen (auch Körperflüssigkeitsbestandteilen) mit der Sensoreinheit zu verringern oder sogar zu verhindern. Auf diese Art kann die Einsatzstabilität der Sensoreinheit gewährleistet werden. Dies geschieht zum Beispiel, indem der Zutritt von Proteasen zur Sensoreineinheit verhindert wird.

Weiterhin kann durch eine geeignete Außenhülle (inklusive gegebenenfalls einer semipermeablen Membran) verhindert werden, dass konkurrierende oder das Nachweissystem störende Moleküle in Kontakt mit der erfindungsgemäßen Sensoreinheit kommen.

Erfindungsgemäß bevorzugt ist es ferner, dass die erfindungsgemäße Sensoreinheit innerhalb des Implantates immobilisiert ist. Dies kann durch eine feste Bindung an das innere der Außenhülle des Implantates geschehen, oder durch eine Bindung an eine Matrix innerhalb des Implantates. Bevorzugt im Rahmen der Erfindung ist, dass die erfindungsgemäße Sensoreinheit mit einem Polymer, bevorzugt Hydrogel innerhalb des Implantates immobilisiert ist. Die Immobilisierung führt zu einer definierten Lage in der Sensoreinheit innerhalb des Implantates, gegebenenfalls zu einer Stabilisierung der erfindungsgemäßen Sensoreinheit. Sie besitzt als weiteren Vorteil, dass selbst bei einer Verletzung der äußeren Hülle des Implantates die Sensoreinheit nicht unkontrolliert in den Körper gelangt. Sofern die Immobilisierung mittels eines Hydrogels erfolgt, besitzt dieses den Vorteil, dass dieses insbesondere für kleine Analyten permeabel ist, so dass ein Kontaktieren dieser Analyten mit der erfindungsgemäßen Sensoreinheit gut gewährleistet werden kann.

Sofern die Sensoreinheit im Implantat einer Matrix immobilisiert ist, ist es selbstverständlich, dass die Matrix so gestaltet ist, dass sie durchlässig für das entsprechende Fluoreszenzlicht ist. Alternativ ist es auch möglich, die Matrix so anzuordnen, dass sie sich nicht zwischen der Sensoreinheit und einem entsprechenden Fluoreszenzdetektor befindet.

Die semipermeable Membran kann im Sinne der vorliegenden Erfindung aus verschiedenen Schichten aufgebaut sein, wobei jede dieser Schichten bestimmte Funktionen übernimmt. Ein stabilisierender Membranteil verhindert bevorzugt das Eindringen von größeren Objekten wie Zellen in das Innere des Implantates, und sorgt bevorzugt dafür, dass die Sensoreinheiten und die Matrix im Inneren verbleiben. Eine weitere Membranschicht sorgt dafür, dass die das Nachweissystem störende und interferierende Teilchen und Moleküle vom Innern des Implantates fern gehalten werden. Eine angiogene Schicht kann zur Ausbildung neuer Blutgefäße in der Nähe des Implantates führen. Die Oberfläche der semipermeablen Membran kann je nach Anwendung hydrophobe oder hydrophile Eigenschaften besitzen, sie kann einen kationischen oder anionischen bzw. metallischen Charakter tragen. Zur Vermeidung von Biofouling können eine Reihe von Modifikationen auf die Membran kovalent bzw. über physikalische Adsorption aufgebracht werden. An die Oberfläche können anorganische oder organische Moleküle über physikalische Adsorption oder kovalente Bindung verknüpft werden, wie z.B. Polymere, Peptide, Proteine, Aptamere, molekular geprägte Polymere, RNA, DNA, siRNA und Nanopartikel. Als typischer Durchmesser der Poren der Membranen (typische Dicke von 0,5 - 10 µm) sind je nach Anwendung Werte im Bereich **1** nm bis **10** µm denkbar, so dass der nachzuweisende Analyt die Membran durchdringen kann, die interferierenden oder das Nachweissystem störenden Teilchen oder Moleküle jedoch nicht.

Alternativ ist auch eine teilweise bioresorbierbare Membran denkbar.

Bevorzugt im Sinne der Erfindung ist ein erfindungsgemäßes Implantat, umfassend an den Außenwänden des Implantates Oberflächengestaltungen oder -modifikationen, die die Biokompatibilität des Implantates verbessern, das Einwachsverhalten steuern und/oder die Thrombogenität reduzieren.

Diese Oberflächenmodifikationen können die gesamte Hülle des Implantates betreffen, ggf. sogar inklusive einer Membran, sofern durch die entsprechende Oberflächenmodifikation nicht die Permeabilität der Membran für den Analyten beseitigt wird. Die Oberflächenfunktionalisierung des Implantates bzw. von Teilen des Implantates können dazu dienen, die Reaktion des Körpers auf das Implantat zu verbessern. Dazu zählen das Einwachsverhalten, die Entzündungsreaktionen, die "Foreign Body Response" und die Vermeidung von Biofouling.

Dabei kann die Oberfläche des Implantates (bzw. ein Teil der Oberfläche) je nach Anwendung hydrophobe oder hydrophile Eigenschaften besitzen. Sie kann sowohl einen kationischen oder anionischen bzw. metallischen Charakter tragen. An die Oberfläche können anorganische oder organische Moleküle über physikalische Adsorption oder kovalente Bindung verknüpft werden, wie z.B. Polymere, Peptide, Proteine, Aptamere, molekular geprägte Polymere, RNA, DNA, siRNA und Nanopartikel. Die Oberfläche kann eine Nano- oder Mikrostrukturierung tragen. Es können zur Strukturierung auf der Oberfläche sowohl runde, sphärische, zylindrische, kegelförmige, quadratische, rechteckige, wie auch längliche Strukturen aufgetragen wie auch abgetragen werden. Dazu zählen Rillen, Röhren, Vollzylinder, Kugeln, Halbkugeln, Quader und Würfel.

Es ist auch eine teilweise bioresorbierbare Oberfläche denkbar.

All diese Ausgestaltungen können je nach geplantem Einsatzort des Implantates innerhalb des Körpers dazu dienen, insbesondere die vorgenannten Reaktionen im Sinne einer verbesserten Bioverträglichkeit des Implantates zu verbessern.

Der Vorteil eines teilweise bioresorbierbaren Implantates (hinsichtlich seiner Außenwand) ist darin zu sehen, dass über die Bioresorption im Bereich des Implantates Mikrobedingungen geschaffen werden, die unerwünschte Körperreaktionen vermeiden helfen und erwünschte Körperreaktionen (wie z.B. ein Einwachsen) begünstigen.

Erfindungsgemäß bevorzugt ist ein Implantat, umfassend eine Fluoreszenzdetektor und eine Vorrichtung zur Übermittlung der durch den Detektor gemessenen Werte aus dem Implantat heraus.

Ein solcher Detektor kann z. B. eine Fotodiode, ein Fotowiderstand, Fototransistor, Photomultiplier oder pyroelektrischer Sensor sein. Bei der Vorrichtung zur Übermittlung der über den Detektor gemessenen Werte kann es sich um die Technologien Surface Acoustic Waves (SAWs), induktive Kopplung und RF-Telemetrie handeln.

Teil der Erfindung ist auch die Verwendung einer erfindungsgemäßen Sensoreinheit oder eines erfindungsgemäßen Implantates zur qualitativen oder quantitativen *in vitro* Bestimmung eines Analyten.

Diese Verwendungsmöglichkeit ist ein besonderer Vorteil der erfindungsgemäßen Sensoreinheit bzw. des erfindungsgemäßen Implantates. Diese sind durch ihren Aufbau in der Lage, über einen verhältnismäßig langen Zeitraum in *in vitro* Daten eines Analyten zu bestimmen. Zur Übermittlung und Auswertung der Daten werden nachfolgend noch Ausführungen gemacht.

Teil der Erfindung ist auch ein Verfahren zur qualitativen oder quantitativen *in vivo* Bestimmung eines Analyten, umfassend die Schritte:
a) Kontaktieren einer Sensoreinheit wie oben beschrieben oder eines Implantates wie oben beschrieben mit einer den Analyt wenigstens potentiell enthaltenden Körperflüssigkeit eines Menschen oder eines Tieres im oder am Körper,
b) Registrieren des durch die Sensoreinheit erzeugten Fluoreszenzsignals
c) Vergleichen des Signals mit einem oder einem Array von Referenzwerten zur qualitativen oder quantitativen Bestimmung des Analyten.

Grundsätzlich ist es möglich, den Schritt b) des erfindungsmäßen Verfahrens im Körper des Menschen oder des Tieres oder außerhalb des Körpers des Menschen oder des Tieres erfolgen zu lassen.

Selbstverständlich ist es entscheidend, dass die Sensoreinheit/das Implantat im oder am Körper so angebracht ist, dass der Analyt potentiell in Kontakt mit der Sensoreinheit treten kann. Dies kann z. B. durch Inkontaktieren des Implantates mit der Blutbahn oder Körperflüssigkeit erfolgen, so dass (auch gegebenenfalls über eine Membran des Implantates) der Analyt in Kontakt mit der Sensoreinheit gelangen kann. Sofern ein solcher Kontakt stattfindet, verändert sich das Fluoreszenzsignal, das von der Sensoreinheit ausgesendet wird. Wie bereits oben angedeutet, kann es sich bei diesen Fluoreszenzsignal beispielsweise um die Intensität (Frequenzdomäne) des Signales oder aber auch um den Verlauf (Zeitdomäne) handeln.

Grundsätzlich sind - wie oben angedeutet - zwei Fälle zu unterscheiden:

### Fall 1)

Das Analysesystem befindet sich außerhalb des Körpers.

Mittels einer Lichtquelle (z.B. Laser) und eines Detektors (Photodiode), die an die optischen Eigenschaften des Nachweissystems angepasst sind (z.B. Wellenlänge, Leistung), werden die optischen Eigenschaften des Nachweissystems in Abhängigkeit von der Analytkonzentration bestimmt. Hierzu wird der erste fluoreszenzaktive Bereich angeregt. Zum einen kann die Fluoreszenz des Donors, des Akzeptors, oder des Donors und Akzeptors bzw. die Fluoreszenzlebenszeit des Donors bestimmt und ausgewertet werden und auf die Konzentration des Analyten in der Messlösung geschlossen werden. Das Implantat enthält somit keine elektronischen Bauteile.

### Fall 2)

Das Analysesystem befindet sich im Implantat.

In Analogie zu Fall 1) werden hier ebenfalls die optischen Eigenschaften in Abhängigkeit von der Analytkonzentration bestimmt (Fluoreszenz des Donors, des Akzeptors, oder des Donors und Akzeptors bzw. die Fluoreszenzlebenszeit des Donors). Diese können durch eine geeignete Elektronik im Implantat ausgewertet werden und über Telemetrie an einen externen Empfänger übertragen werden. Die Energieversorgung (Batterie) liefert für den Nachweis und die Datenübertragung die nötige Energie. Es ist ebenfalls eine Ausführung mit einer Energieübertragung von außen sinnvoll.

Die Auswertung der Messwerte, die in beiden Fällen gewonnen werden, erfolgt durch Vergleichen des jeweiligen Signals mit einer Kalibrationskurve. Dieser Abgleich kann selbstverständlich automatisch erfolgen. Die Kalibrationskurve muss so ermittelt werden, dass unter den Einsatzbedingungen vergleichbaren Bedingungen Signale in Abhängigkeit von unterschiedlichen Konzentrationen des jeweiligen Analyten erzeugt werden. Dies ist z. B. denkbar, für den Fall einer implantierten Glukosekonzentrationsmesseinheit indem Signale in Abhängigkeit von unterschiedlichen Glukosekonzentrationen erzeugt werden und diese Signale durch eine Haut *in vitro* hindurchgeführt werden, wobei die Dicke dieser Haut der Implantationstiefe entspricht. Sinnvollerweise werden die Glukosekonzentrationsermittlungen auch in einem vergleichbaren System (wie z.B. Blut/interstitielle Flüssigkeit) erzeugt.

Durch den Abgleich mit der entsprechenden Kalibrationskurve ist es somit möglich, unmittelbar, also gegebenenfalls in Echtzeit auf den physiologischen Zustand des Menschen/des Tieres rückzuschließen. Die Kalibrationskurve wird durch Analyse mehrerer Referenzlösungen ermittelt, die den Analyten im physiologisch relevanten Bereich enthält und das entsprechende Messsignal (Fluoreszenz bzw. Fluoreszenzlebenszeit) ermittelt wird.

In Abhängigkeit von dem jeweiligen *in vitro* Auswertungsergebnis können dann Maßnahmen ergriffen werden. So ist es z.B. möglich, im Falle einer Hyperglykämie eine (erhöhte) Insulingabe durch eine gegebenenfalls gekoppelte Insulinpumpe auszulösen. Im Falle einer Hypoglykämie könnte dagegen die Insulingabe gedrosselt werden. Auf diese Art können Morbidität und Mortalität von Patienten mit Diabetes Mellitus reduziert werden.

Entsprechendes gilt auch für die Reaktion auf die Messdaten anderer Analyten wie z.B. von Kalzium und bei diesem Analyten gekoppelten Krankheitsbildern.

Ein weiterer Vorteil des erfindungsgemäßen Implantates ist darin zu sehen, dass durch eine geeignete Ausgestaltung eine hohe Stabilität der Sensoreinheit erreicht werden kann. Damit ist es möglich, Sensoreinheiten im Sinne eines langzeitstabilen Systems zur Konzentrationsbestimmung des jeweiligen Analytens herzustellen und zum Einsatz zu bringen.

Figurenbeschreibung
- Fig. 1A, 1B: stellt eine erfindungsgemäße Sensoreinheit dar, deren Messsignal durch FRET beeinflusst wird.
- Fig. 2A, 2B: stellt eine erfindungsgemäße Sensoreinheit dar, deren Messsignal durch Quench beeinflusst wird.

Es sei noch einmal darauf hingewiesen, dass allgemein das Messsignal z.B. die Intensität oder die Lebenszeit der Fluoreszenz sein kann.

In den Figuren haben die Bezugszeichen folgende Bedeutung
1 erster fluoreszenzaktiver Bereich (Fluorophor)
3 Verbindungsbereich
5 zweiter fluoreszenzaktiver Bereich (Fluorophor)
7 Analyt
9 zweiter fluoreszenzaktiver Bereich (Quencher)

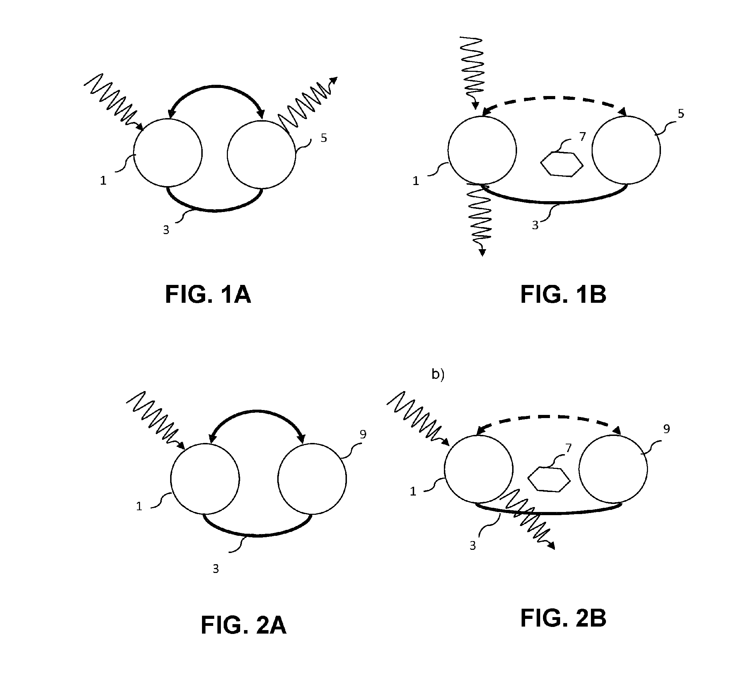

In der Figur 1A ist die Situation gezeigt, in der ohne Analyt 7 am Verbindungsbereich 3 der erste fluoreszenzaktive Bereich 1 (Fluorophor) und der zweite fluoreszenzaktive Bereich 5 (Fluorophor) in räumlicher Nähe zueinander stehen, so dass ein Fluoreszenzresonanzenergietransfer (FRET) stattfindet. Durch das Bindungsereignis des Analyten 7 an den Verbindungsbereich 3 (Figur 1B) vergrößert sich der Abstand des ersten fluoreszenzaktiven Bereiches 1 zum zweiten fluoreszenzaktiven Bereich 5, so dass kein oder nur noch ein verringerter FRET auftritt. Damit verändert sich das Fluoreszenzsignal.

In der Figur 2 ist im Falle des Figur 2A der erste fluoreszenzaktive Bereich 1 (Fluorophor) in räumlicher Nähe zum zweiten Fluoreszenzaktiven Bereich 9 (Quencher), so dass wenigstens einen Teil der Fluoreszenz gelöscht wird. In der Figur 2B hat sich die räumliche Entfernung der beiden fluoreszenzaktiven Bereiche zueinander durch das Bindungsereignis des Analyten 7 an den Verbindungsbereich 3 vergrößert. Dadurch verringert sich die Fluoreszenzlöschung (Quench) und das Fluoreszenzsignal verändert sich in Abhängigkeit vom Bindungsereignis.

### Beispiel Glukosesensor:

Für die optische Konzentrationsbestimmung von Glukose in Körperflüssigkeiten werden an das Glukosebindungsprotein (GBP) des E. coli Stamms jeweils am N-Terminus ein fluoreszenter Nanopartikel (Nanophosphor, YAG:Ce3⁺) und an einer anderen von der ersten räumlich getrennten Position ein Fluoreszenzquencher (Goldnanopartikel) gebunden. Der Abstand des fluoreszierenden Nanopartikels zum Quenchner beträgt ca. 4 nm. Alternativ zum Quencher kann auch ein weiterer fluoreszenter Nanopartikel (Nanophosphor oder Quantum Dot) angebracht werden.

Zur Bindung an das sensitive Molekül (Verbindungsbereich, hier GBP) wird die Oberfläche des fluoreszenten Nanopartikels durch Anwendung von APTMS (3-Aminopropyltrimethoxysilan) und 1,4-BD (1,4-Butandiol) so funktionalisiert, dass sie durch Zugabe von SB (Sulfosuccinimidyl-6-(biotinamido)-hexanoat biotinylierendes Agens) mit einer Biotinfunktion versehen wird. Das GBP wird am N-Terminus über eine biotechnologische Modifikation (rekombinant) mit einem monovalenten Streptavidin (trägt nur eine Biotinbindungstasche) versehen. Dabei werden die optischen Eigenschaften nicht verändert. Diese Vorgehensweise ist angelehnt an Azakure et al., Anal Bioanal Chem (2006) 386:1641-1647 "Tagging of avidin immobilized beads with biotinylated YAG:Ce3+ nanocrystal phosphor".

Der Fluoreszenzquencher (Goldnanopartikel) wird wie folgt mit dem C-Terminus des GBP verbunden: An den C-Terminus wird ein Cystein-Tag bestehend aus 5 Cysteinen (gemeinsam mit der rekombinanten Modifikation des N-Terminus) angefügt. Die Goldnanopartikel werden mit einer Mischung aus mPEG-SH und HS-PEG-NH2 (PEG = poly(ethylene glycol)) inkubiert. Nach Zugabe des Crosslinkers (4-(N-maleimidomethyl)cyclohexane-1-carboxyl-säure-3-sulfo-N-hydroxysuccinimid-ester-Natriumsalz) werden die Nanopartikel mit den N-terminalen Thiolgruppen des GBP kovalent verbunden. Diese Vorgehensweise ist angelehnt an Li et al., Biochemical and Biophysical Research Communications 355 (2007) 488-493, "Immobilization of glucose oxidase onto gold nanoparticles with enhanced thermostability", jedoch hat sich abweichend im Zusammenhang mit der Erfindung ein Cystein-Tag mit n ≥ 1 als besonders effektiv herausgestellt.

Das Gesamtmolekül wird durch Vereinigung des Streptavidin-GBP-Goldnanopartikels mit dem biotinylierten fluoreszenten Nanopartikel erzeugt

Funktionsweise des Sensors:

### Fall 1)

In der glukosefreien Form stellt GBP eine relativ kompakte Struktur dar, der Abstand des fluoreszierenden Nanopartikels und des Fluoreszenzquenchers zueinander ist klein (Abstand ca. 3 nm). Dadurch wird dieFluoreszenz gelöscht. Binden nun mehr und mehr Glukosemoleküle an den Glukoserezeptor, kommt es bei den Rezeptoren zur Konformationsänderung, was sich durch einen Anstieg der Gesamtfluoreszenz (verminderter Quench) bemerkbar macht. Die Fluoreszenzlebenszeit weist veränderte Werte auf.

### Fall 2)

Im Falle der oben beschriebenen Alterative können analog zwei fluoreszente Nanopartikel (Donor und Akzeptor (siehe oben)) an den Glukoserezeptor (Verbindungsbereich) angebracht werden. Im glukosefreien Zustand sind die beiden fluoreszenten Partikel relativ nah beieinander (Abstand beträgt ca. 6 nm) lokalisiert. Hierduch kann die Energie strahlungslos vom Donor zum Akzeptor über Resonanz-Energie-Transfer übertragen werden. Die Fluoreszenz des Akzeptors ist groß. Bindet Glukose an das GBP, vergrößert sich der Abstand zwischen Donor und Akzeptor, die Fluoreszenz des Akzeptors wird vermindert, die Fluoreszenzlebenszeit weist veränderte Werte auf.

Für den Einsatz *in vivo* wird die Konzentration der beschriebenen Sensoreinheiten so gewählt, dass bei physiologisch relevanten Konzentrationen von Glukose nicht alle Sensoren abgesättigt sind. Typische Glukosekonzentrationen sind hierbei 0,1 bis 25 mM Glukose.

Die Glukosekonzentration wird im vorliegenden Beispiel durch den Vergleich mit einer Kalibrationskurve ermittelt. Hierzu wird der physiologisch relevante Bereich von beispielsweise 0 ― 25 mM Glukose in Abhängigkeit von der Glukosekonzentration mittels Referenzlösungen (z.B. 0, 5, 10, 15, 20, 25 mM Glukose) die zugehörige Fluoreszenzintensität bzw. Fluoreszenzlebenszeit ermittelt.

Durch Abgleich des jeweiligen Messwertes gegen die Kalibrationskurve ist es somit möglich mittels der Beispielsensoreinheit (bzw. einer Vielzahl dieser Sensoreinheiten) die *in situ* Konzantration des Analyten zu ermitteln.

## Patentansprüche

1. Im Implantat einsatzstabile Sensoreinheit zum qualitativen und/oder quantitativen *in vivo* Bestimmung eines Analyten umfassend
(i) einen Verbindungsbereich (3) mit wenigstens einer Bindungsstelle für den Analyten (7), wobei der Verbindungsbereich im Falle der Bindung des Analyten an die Bindungsstelle eine räumliche Veränderung erfährt,
(ii) einen ersten fluoreszenzaktiven Bereich (1) und
(iii) einen zweiten fluoreszenzaktiven Bereich (5/9), wobei der erste fluoreszenzaktive Bereich (1) und der zweite fluoreszenzaktive Bereich (5/9) an den Verbindungsbereich (3) so gebunden sind, dass im Falle einer durch die Bindung des Analyten an den Verbindungsbereich (3) bewirkte räumliche Veränderung des Verbindungsbereiches (3) eine Veränderung des Abstandes des erste fluoreszenzaktiven Bereiches (1) und des zweiten fluoreszenzaktiven Bereiches (5/9) zueinander erfolgt, ohne dass die Bindungen der Fluoreszenzaktiven Bereiche (1, 5/9) an den Verbindungsbereich gelöst werden und wobei einer oder beide der fluoreszenzaktiven Bereiche (1, 5/9) ≥ 60 Gew.% aus einem anorganischen Material besteht.

2. Sensoreinheit nach Anspruch 1, wobei einer oder beide der fluoreszenzaktiven Bereiche (1, 5/9) ein gegebenenfalls beschichteter fluoreszenzaktiver Nanopartikel sind.

3. Sensoreinheit nach Anspruch 2, wobei der Nanopartikel ausgewählt ist aus der Gruppe bestehend aus Quantum Dot, Nanophosphor und farbstoffmarkiertem Silikat.

4. Sensoreinheit nach einem der Ansprüche 1 bis 3, wobei der Analyt (7) ausgewählt ist aus der Gruppe bestehend aus Elektrolyten, Kohlehydraten, Metaboliten, Stoffwechselprodukten, Aminosäuren, Peptiden, Fetten, Fettsäuren, Lipiden, Proteinen, Neurotransmitter, Polyelektrolyten, Ribonukleinsäuren, Desoxyribonukleinsäuren, Nukleotiden, Hormonen und Wirkstoffen.

5. Sensoreinheit nach Anspruch 4, wobei der Analyt (7) Glukose, Kalium, Kalzium, ein Peptid oder Protein ist.

6. Implantat, umfassend eines Sensoreinheit nach einem der Ansprüche 1 bis 5.

7. Implantat nach Anspruch 6, umfassend eine für den Analyten (7) durchlässige Membran.

8. Implantat nach Anspruch 6 oder 7, wobei die Sensoreinheit innerhalb des Implantates immobilisiert ist.

9. Implantat nach Anspruch 8, wobei die Sensoreinheit in einer Matrix oder an den Innenwänden des Implantates immobilisiert ist.

10. Implantat nach einem der Ansprüche 5 bis 9, umfassend einen Fluoreszenzdetektor und eine Vorrichtung zur Übermittlung der durch den Detektor gemessenen Werte aus dem Implantat heraus.

11. Implantat nach einem der Ansprüche 6 bis 10, umfassend auf den Außenwänden des Implantates Oberflächengestaltungen oder ―modifikationen, die die Biokompatibilität des Implantates verbessern, das Einwachsverhalten steuern und/oder die Thrombogenität reduzieren.

12. Implantat nach Anspruch 11, wobei ein Teil der Außenwand des Implantates bioresorbierbar ist.

13. Verwendung eine einer Sensoreinheit nach einem der Ansprüche 1 bis 5 oder eines Implantates nach einem der Ansprüche 6 bis 11 zur qualitativen oder quantitativen *in vivo* Bestimmung eines Analyten.

14. Verfahren zur qualitativen oder quantitativen *in vivo ―* Bestimmung eines Analyten, umfassend die Schritte:
a) Kontaktieren einer Sensoreinheit nach einem der Ansprüche 1 bis 5 oder eines Implantates nach einem der Ansprüche 6 bis 11 mit einer den Analyt wenigstens potentiell enthaltenden Körperflüssigkeit eines Menschen oder eines Tieres im oder am Körper,
b) Registrieren des durch die Sensoreinheit erzeugten Fluoreszenzsignals
c) Vergleichen des Signals mit einem oder einem Array von Referenzwerten zur qualitativen oder quantitativen Bestimmung des Analyten.

15. Verfahren nach Anspruch 14, wobei Schritt b) (i) im Körper des Menschen oder des Tieres oder (ii) außerhalb des Körpers des Menschen oder des Tieres erfolgt.
